# EUROPÄISCHE PATENTSCHRIFT

(11) **EP 2 010 894 B1**
(45) Veröffentlichungstag und Bekanntmachung des Hinweises auf die Patenterteilung: **18.04.2012**
(21) Anmeldenummer: 07724409.3
(22) Anmeldetag: 20.04.2007
(51) Int. Cl.: G01N 21/78, G01N 33/00

(54) **WASSERSTOFFSENSOR**
HYDROGEN SENSOR
CAPTEUR D'HYDROGÈNE

(30) Priorität: 20.04.2006 DE 102006018767
(43) Veröffentlichungstag der Anmeldung: 07.01.2009
(73) Patentinhaber: Wienecke, Marion, 23966 Wismar (DE)
(72) Erfinder: WIENECKE, Marion, 23966 Wismar (DE); FEDTKE, Petra, 23992 Rügkamp (DE); BARFELS, Torsten, 23970 Wismar (DE); BUSCH, Andreas, 21075 Hamburg (DE); BUNESCU, Carmen, 26871 Papenburg-Aschendorf (DE); BRAMANN, Gero, 23966 Wismar (DE)
(74) Vertreter: Seemann, Ralph
(86) Internationale Anmeldenummer: PCT/EP2007/003472
(87) Internationale Veröffentlichungsnummer: WO 2007/121935

(56) Entgegenhaltungen:
- EP-A- 0 120 231
- WO-A-95/30889
- US-A- 4 661 320
- US-A1- 2005 089 260
- US-A1- 2005 169 807
- US-B1- 6 535 658
- FEDTKE P ET AL: "Hydrogen sensor based on optical and electrical switching" SENSORS AND ACTUATORS B, ELSEVIER SEQUOIA S.A., LAUSANNE, CH, Bd. 100, Nr. 1-2, 1. Juni 2004 (2004-06-01), Seiten 151-157, XP004509067 ISSN: 0925-4005 in der Anmeldung erwähnt

## Beschreibung

Die Erfindung betrifft einen Wasserstoffsensor mit einer Strahlenquelle, mittels der elektromagnetische Strahlen auf ein Sensormedium gestrahlt werden, wobei das Sensormedium einen Transmissionskoeffizienten hat, der in Abhängigkeit der Konzentration von Wasserstoff in der Umgebung des Sensormediums variiert, und mit einem Detektor, der wenigstens einen Teil der durch das Sensormedium transmittierten Strahlen detektiert.

Das physikalische Prinzip eines entsprechenden Sensors ist aus Petra Fedtke, Marion Wienecke, Mihaela-C. Bunescu, Marlis Pietrzak, K. Deistung, Erika Borchardt in "Hydrogen sensor based on optical and electrical switching", Sensors and Actuators B 100 (2004) 151-157, bekannt. Hierbei dient eine dünne Palladiumschicht bzw. eine Schicht aus einer Palladiumclusterschicht dazu, den physikalischen Effekt auszunutzen, dass die optische Transmission der Palladiumschicht sich bei Anwesenheit von Wasserstoff in der Umgebung der Palladiumschicht ändert. Dies geschieht durch Einlagerung bzw. chemische Reaktion von Wasserstoff mit dem Palladium, wodurch sich die elektronische Struktur des Palladiums, insbesondere um die Fermikante herum, ändert.

US 6 535 658 B1 betrifft einen Wasserstoffsensor auf der Grundlage eines porösen Glasstabs. Der poröse Glasstab bildet eine Glasmatrix, die nach ihrer Herstellung zunächst mit Wolframoxid behandelt wird, wobei Wolframoxid an Sauerstoffatome in den Poren des Glases gebunden wird, und anschließend mit Palladium, das durch eine Hitzebehandlung bei 500 °C so behandelt wird, dass sich ein Palladium-Überzug bzw. eine Palladium-Schicht auf der Oberfläche der Poren des Glasstabes bildet.

In US 4 661 320 A ist ein Gassensor mit einer Einrichtung offenbart, in der in Gegenwart von Wasserstoff die Lichtabsorption geändert wird. Eine Einrichtung, die den Sensor verwendet, hat die Form eines Laminats aus einem Metall, das Wasserstoff oder das Gas einer Wasserstoff enthaltenden Verbindung absorbiert und dissoziiert, und einer festen Verbindung, die unter Änderung der Lichtabsorption durch die Wasserstoffatome, die in dem Metall gebildet werden, reduziert wird.

US 2005/169807 A1 betrifft einen optischen Transmissionswasserstoffsensor mit einem dünnen Film aus wasserstoffsensitivem Material aus einer Palladiumlegierung, beispielsweise einer Palladium/Gold-Legierung. Die dünne Schicht weist eine nanokristalline Struktur auf.

EP 0 120 231 A offenbart eine Vorrichtung für den Gasnachweis mit einem aus Metalloxid bestehenden Sensor, wobei Oxide oder Oxidmischungen einer Vielzahl von Elementen verwendet werden.

Es ist Aufgabe der vorliegenden Erfindung, den bekannten physikalischen Effekt für einen, insbesondere portablen, Wasserstoffsensor auszunutzen und derart weiter zu bilden, dass ein sehr effizienter Wasserstoffsensor angegeben wird, dessen Empfindlichkeit hoch ist. Ferner soll ein alternativer Wasserstoffsensor angegeben werden.

Gelöst wird diese Aufgabe durch einen Wasserstoffsensor mit einer Strahlenquelle, mittels der elektromagnetische Strahlen auf ein Sensormedium gestrahlt werden, wobei das Sensormedium einen Transmissionskoeffizienten aufweist, der in Abhängigkeit der Konzentration von Wasserstoff in der Umgebung des Sensormediums variiert, und mit einem Detektor, der wenigstens einen Teil der durch das Sensormedium transmittierten Strahlen detektiert, wobei das Sensormedium Cluster enthaltend oder bestehend aus einer Palladiumlegierung, Yttrium, Scandium, wenigstens einem Lanthanid, wenigstens einem Actinoid, Wolframoxid und/oder Vanadiumoxid und/oder eine Mischung oder Verbindung dieser Materialien aufweist, wobei die Cluster in einer Matrix aus einem Polymer eingebettet sind.

Ein besonders haltbares Sensormedium, das einen hohen Anteil an Wasserstoff aufnehmen kann, ohne Schäden aufzuweisen, liegt vor, da das Sensormedium Cluster aus Palladiumlegierungen, Yttrium, Scandium, wenigstens ein Lanthanid, wenigstens ein Actinoid, Wolframoxid und/oder Vanadiumoxid und/oder eine Mischung oder Verbindung dieser Materialien aufweist. Als Palladiumlegierungen dienen insbesondere Palladiumlegierungen mit Eisen, Nickel und Wolfram. Die Lanthanide sind die Elemente 57 bis 71 des Periodensystems der Elemente. Die Actinoide sind die Elemente 89 bis 103 des Periodensystems der Elemente.

Cluster sind im Rahmen der Erfindung insbesondere Ansammlungen von miteinander chemisch oder physikalisch gebundenen Atomen der eben genannten Materialien. Cluster können insbesondere auch Kristalle oder Kristallite sein. Das Sensormedium ist ein Komposit aus Clustern und einem weiteren Material, in das die Cluster eingebettet sind, nämlich einer Matrix aus einem Polymer. Hierdurch können die Änderungen der Gitterkonstanten des Materials, aus dem die Cluster bestehen bzw. das die Cluster enthalten, bei Vorliegen von verschiedenen Wasserstoffkonzentrationen ausgeglichen werden, so dass das Sensormedium dann nicht bei zu hohem Anteil von Wasserstoff in dem Sensormedium bzw. bei wiederholter Beladung mit Wasserstoff degradiert bzw. irreversibel zerstört wird.

Vorzugsweise liegt das Wolframoxid in einer chemischen Formel WO₃ vor. Vorzugsweise liegt Vanadiumoxid in einer chemischen Formel V₂O₅ vor. Die Materialien bilden vorzugsweise mit Wasserstoff Metallhydride, wie beispielsweise Metall-H₃.

Durch Verwendung von Clustern enthaltend die bzw. bestehend aus den oben genannten Materialien wird ein Wasserstoffsensor mit einer hohen Lebensdauer und einer hohen Langzeitstabilität ermöglicht.

Erfindungsgemäß sind die Cluster in einer Matrix aus einem Polymer eingebettet. Vorzugsweise haben die Cluster einen Durchmesser von 1 nm bis 30 nm, insbesondere 2 nm bis 15 nm, insbesondere 3 nm bis 10 nm, vorzugsweise bis 3 bis 5 nm. Die Cluster sind vorzugsweise Nanokristallite bzw. Nanokristalle.

Das Polymer umfasst oder besteht vorzugsweise aus Polytetrafluorethylen, Polyterephthalat, Polyimid, Polymethylmethacrylat und/oder Polycarbonat.

Vorzugsweise ist das Sensormedium auf einem Substrat aufgebracht. Hierdurch wird die Handhabbarkeit des Sensormediums verbessert.

Wenn zwischen dem Substrat und dem Sensormedium eine Pufferschicht vorgesehen ist, können Differenzen zu der Gitterkonstanten zwischen Substrat und dem Sensormedium kompensiert werden. Beispielsweise eignet sich als Pufferschicht Calciumfluorid (CaF₂). Das Substrat ist vorzugsweise für die elektromagnetischen Strahlen durchsichtig bzw. im Wesentlichen durchsichtig und vorzugsweise nicht reaktiv auf Wasserstoff.

Wenn vorzugsweise auf dem Sensormedium eine Deckschicht vorgesehen ist, die insbesondere vorzugsweise durchsichtig, nicht mit Wasserstoff reagierend oder im Wesentlichen nicht mit Wasserstoff reagierend und/oder wasserstoffdurchlässig ist, ist das Sensormedium in dem Sensor auch geschützt angeordnet. Die Deckschicht kann auch teilweise reflektierend ausgebildet sein, so dass ein nicht nur zweifacher Durchtritt der elektromagnetischen Strahlen durch das Sensormedium erzeugt werden kann, sondern auch ein mehrfacher Durchtritt. Vorzugsweise ist das Sensormedium als Schicht ausgebildet. Wenn auf dem Sensormedium eine Deckschicht vorgesehen ist, kann diese zum Schutz des Sensormediums dienen und/oder auch reflektierend ausgebildet sein, um zu ermöglichen, dass die elektromagnetischen Strahlen mehrfach durch das Sensormedium transmittiert werden können. Als Deckschicht kann im Rahmen der Erfindung insbesondere Platin, Gold oder ein anderes inertes Material dienen. Die Dicke der Deckschicht sollte in diesem Fall so dünn sein, dass Wasserstoff ohne Probleme hindurch diffundieren kann und auch die elektromagnetischen Strahlen, die vorzugsweise im Bereich des sichtbaren oder ultravioletten Lichts sind, wenigstens abschnittsweise hindurchtreten können. Vorzugsweise ist an der Stelle, an der die elektromagnetischen Strahlen auf das Sensormedium das erste Mal treffen, keine Deckschicht oder eine für diese Strahlen durchsichtige Deckschicht vorgesehen. Diese Strahlen werden dann vorzugsweise durch das Sensormedium transmittiert, gelangen an einen Spiegel bzw. Reflektor, werden reflektiert und wieder durch das Sensormedium geleitet zu einer Deckschicht, die für die elektromagnetischen Strahlen reflektierend ist, um erneut durch das Sensormedium hindurchgeleitet zu werden, noch einmal reflektiert und noch einmal durch das Sensormedium transportiert zu werden, um dann aus dem Sensormedium auszutreten und zu einem Detektor geleitet zu werden. Hierdurch kann das Sensormedium im Verhältnis zur Dicke des Sensormediums viermal so empfindlich sein wie herkömmliche Sensormedien bzw. Sensoren bei gleicher Dicke. Vorzugsweise ist das Sensormedium als Schicht ausgebildet.

Vorzugsweise ist ein Reflektor vorgesehen, der die durch das Sensormedium transmittierten Strahlen zu dem Sensormedium zurück reflektiert. Der erfindungsgemäße Wasserstoffsensor nutzt hierbei die grundsätzliche Idee, das Sensormedium wenigstens zweimal zu nutzen, um so bei wenig Materialaufwand, der zu stabilen Sensormedien führt, eine große Empfindlichkeit des Sensors zu ermöglichen und eine hohe Lebensdauer. Gemäß dem Stand der Technik ist es nämlich so, dass entsprechend dicke Schichten, üblicherweise aus Palladium oder Palladiumclustern Verwendung finden, die ab einer entsprechend hohen Beladung mit Wasserstoff, beispielsweise von über 10% bei den Palladiumclustern und von über 4% bei kristallinen Schichten nach einigen Beladungszyklen mit Wasserstoff sich von dem Substrat ablösen, so dass der Sensor keine reproduzierbaren Ergebnisse mehr zur Verfügung stellt. Durch die erfindungsgemäße Lösung, die elektromagnetischen Strahlen wenigstens zweimal durch das Sensormedium laufen zu lassen, kann ein sehr dünnes Sensormedium Verwendung finden, was dann nicht zu einer entsprechenden Degradation des Sensormediums aufgrund häufigerer Beladung mit Wasserstoff oder aufgrund hoher Konzentration von Wasserstoff in der Umgebung des Sensormediums führt.

Vorzugsweise ist das Substrat der Reflektor oder es ist ein Reflektor auf dem Substrat aufgebracht. Hierzu ist insbesondere vorzugsweise auf der Oberseite des Substrats das Sensormedium aufgebracht und auf der Unterseite bzw. Rückseite eine spiegelnde Fläche aufgebracht. Das Substrat kann beispielsweise Glas bzw. ein Spiegel sein.

Ein sehr gut handhabbarer Wasserstoffsensor ist dann gegeben, wenn die elektromagnetischen Strahlen mit wenigstens einem ersten Lichtwellenleiter von einer Strahlenquelle zu dem Sensormedium geleitet werden. Vorzugsweise werden die von dem Reflektor reflektierten und durch das Sensormedium transmittierten Strahlen mit wenigstens einem zweiten Lichtwellenleiter zu dem Detektor geleitet. Der wenigstens eine erste und der wenigstens eine zweite Lichtwellenleiter verlaufen vorzugsweise, wenigstens abschnittsweise im Wesentlichen parallel bzw. sind dergestalt angeordnet. Vorzugsweise verlaufen die Lichtwellenleiter im Wesentlichen vollständig über die gesamte Länge parallel zueinander.

Vorzugsweise ist am zu dem Sensormedium gerichteten Ende des wenigstens einen ersten und/oder zweiten Lichtwellenleiters eine die Strahlen bündelnde Linse vorgesehen. Der Fokus der Linse ist vorzugsweise auf die Spiegelfläche oder das Sensormedium eingestellt.

Die Erfindung wird nachstehend ohne Beschränkung des allgemeinen Erfindungsgedankens anhand von Ausführungsbeispielen unter Bezugnahme auf die Zeichnungen beschrieben. Bezüglich aller im Text nicht näher erläuterten erfindungsgemäßen Einzelheiten wird ausdrücklich auf die Zeichnungen verwiesen. Es zeigen:
- Fig. 1: eine schematische Schnittdarstellung durch einen erfindungsgemäßen Sensor,
- Fig. 2: eine schematische Schnittdarstellung einer durch ein Substrat mit entsprechend aufgebrachten Schichten,
- Fig. 3: eine Transmissionselektronenmikroskopaufnahme einer Sensorschicht.

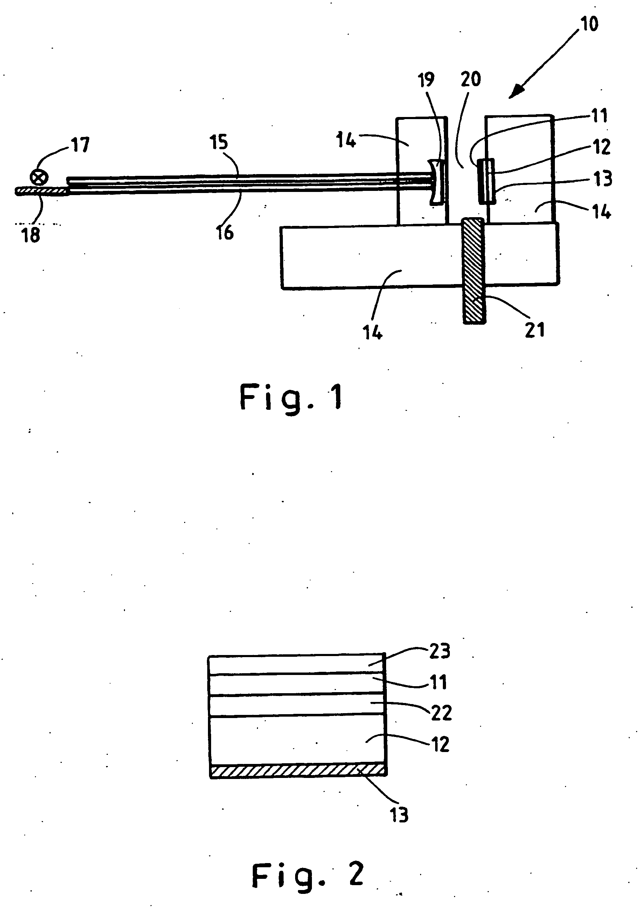

Fig. 1 zeigt eine schematische Schnittdarstellung durch einen erfindungsgemäßen Sensor 10. Das Messprinzip des Sensors beruht auf der Messung der Intensitätsänderung eines Lichtsignals. Das Signal bzw. die Strahlen werden mittels einer Leuchtdiode bzw. Lichtquelle 17, vorzugsweise mit einer Wellenlänge von 350 bis 450 nm erzeugt. Das Licht, das vorzugsweise im Ultravioletten liegt, wird direkt in einen Ultraviolett-Lichtwellenleiter 15 mit, insbesondere 400 µm Durchmesser, eingekoppelt. Durch den Lichtwellenleiter 15 werden die Strahlen bzw. das Lichtsignal zur Messstelle transportiert. Vorzugsweise werden die Strahlen senkrecht auf die Funktionsschicht bzw. Sensorschicht 11 geleitet. Hierzu kann eine Kollimatorlinse 19 dienen. Bei Verwendung einer Kollimatorlinse ist das spätere Messsignal bei gleicher Lichtleistung höher. Die Anordnung funktioniert allerdings auch ohne Kollimatorlinse 19, da insbesondere die Einkopplung des reflektierten Lichtes in den zum Detektor 18 führenden Lichtwellenleiter 15 auf der Streuung des Lichtes an der nanostrukturierten Sensorschicht 11 beruht.

Wird eine Kollimatorlinse 19 verwendet, ist es nicht notwendig, diese zu justieren, um den reflektierten Strahl in den Lichtwellenleiter 15 einzukoppeln. Zwischen der Kollimatorlinse 19 bzw. in der Variante ohne Kollimatorlinse zwischen dem Lichtwellenleiter 15 bzw. 16 und der Sensorschicht 11 ist das zu messende Medium in der Umgebung 20 angeordnet. Der Abstand zwischen Kollimatorlinse 19 bzw. der Lichtwellenleiter 15, 16 zu der Sensorschicht 11 beträgt wenige Millimeter. Die Strahlen durchdringen bzw. treten durch die Sensorschicht 11 und werden je nach Wasserstoffgehalt entsprechend in der Stärke bzw. Leistung beeinflusst. Je mehr Wasserstoff in der Umgebung 20 vorgesehen ist, um so mehr Wasserstoff kann in die Sensorschicht 11 eingelagert werden und die aktive Substanz, insbesondere chemochromatische Substanz, dort derart ändern, dass der Transmissionskoeffizient verringert wird. Das heißt, das durch die Sensorschicht transmittierte Licht wird in Abhängigkeit der Konzentration von Wasserstoff gedämpft.

Die Sensorschicht 11 ist vorzugsweise auf einem Glassubstrat oder einem Substrat 12, wie beispielsweise einem Saphiersubstrat oder einem Strontiumtitanatsubstrat oder einem Diamantsubstrat aufgebracht. Das Substrat 12 ist mit einer reflektierenden Rückseite 13, also einer Spiegelfläche 13, versehen, wodurch die Strahlen reflektiert werden und erneut durch die Sensorschicht 11 treten. Dies führt zu einer erneuten Dämpfung der Strahlen, die durch eine Kollimatorlinse 19 in den zweiten Lichtwellenleiter 16 eingekoppelt werden. An dem der Sensorschicht abgewandten Ende des Lichtwellenleiters 16 ist ein Detektor in Form einer Silicium-Photodiode vorgesehen, die die optischen Strahlen bzw. das optische Signal in ein elektrisches Signal wandelt. Die entsprechenden Komponenten sind auf einer Halterung 14 befestigt. Außerdem ist eine Gaszufuhrleitung 21 vorgesehen, die für die Zufuhr des möglicherweise mit Wasserstoff versehenen Gases sorgt. In Fig. 1 ist der Sensor offen dargestellt. Dieser ist üblicherweise in einem nicht dargestellten geschlossenen Gehäuse mit Ausnahme der Gaszufuhrleitung 21 vorgesehen. Zur besseren Durchströmung des Sensors kann auch eine zweite Öffnung bzw. eine zweite Gaszufuhrleitung 21 vorgesehen sein.

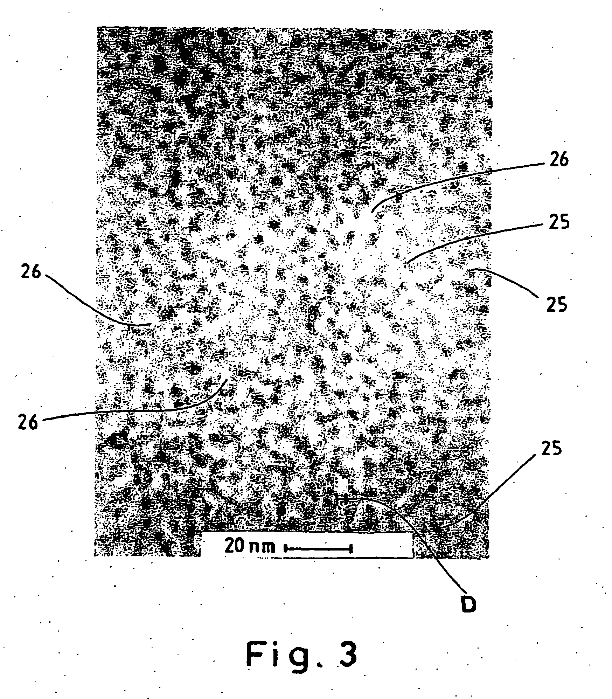

Die Sensorschicht 11 ist in diesem Ausführungsbeispiel 40 nm oder 50 nm dick. Sie kann vorzugsweise auch 10 nm bis 30 nm dick sein. Die Fläche der Sensorschicht 11 ist vorzugsweise wenigstens so groß wie die Strahlfläche der eingekoppelten Strahlen. Die Sensorschicht besteht vorzugsweise aus einem Nanokomposit umfassend Cluster 25 (vgl. Fig. 3) in einer Größe von 1 nm bis 30 nm, insbesondere bei ca. 5 nm. In Fig. 3 ist eine Transmissionselektronenmikroskopaufnahme der Sensorschicht 11 dargestellt. Die dunklen Bereiche sind die metallischen Bereiche bestehend beispielsweise aus Palladiumlegierung, Yttrium, Scandium, einem Lanthanid, einem Actinoid, Wolframoxid und/oder Vanadiumoxid. Die grauen bzw. helleren Bereiche bestehen aus einem Polymer oder einer Mischung aus Polymeren, die eine Art Matrix bilden, in die die Cluster eingebettet sind. Im unteren Bereich der Fig. 3 ist eine Grö-βenangabe vorgesehen. Der dort angegebene weiße Strich vor dem schwarzen Hintergrund hat eine Länge von 20 nm. D soll den ungefähren Durchmesser eines Clusters darstellen und beträgt für den dargestellten Cluster ca. 4 nm. In diesem Beipiel, das nicht unter die Erfindung fällt, ist das Polymer Polytetrafluorethylen. Die Nanokristalle bzw. Cluster 25 sind aus Palladium. Die Größe der Cluster kann durch Tempern bei Temperaturen bis unter 300° C variiert werden.

Fig. 2 zeigt eine Schnittdarstellung durch ein Substrat mit entsprechend aufgebrachten Schichten. Das Substrat 12 hat auf dessen Unterseite eine Spiegelfläche 13 vorgesehen. Auf der Oberseite des Substrats 12 ist eine Pufferschicht 22, beispielsweise eine Calciumfluoridschicht. Hierauf ist die Nanokompositschicht 11 bzw. Sensorschicht 11 aufgebracht und darauf wiederum eine Deckschicht 23, beispielsweise aus Platin.

Die Schichten können auf dem Substrat durch Sputtern, beispielsweise Magnetron-Sputtern oder mit Laserablation, mit PECVD, einem Sol-Gel-Verfahren oder mit Spin-Coating aufgebracht werden.

Durch die wenigstens doppelte Transmission der Strahlen durch die Sensorschicht 11 können auch mit dünnen Schichten signifikante Signalveränderungen bei Vorliegen von entsprechenden Wasserstoffkonzentrationen erzeugt werden. Selbst hohe Wasserstoffkonzentrationen führen bei sehr dünnen Schichten nicht zu einer Degradation der Sensorschicht 11 bzw. zu einer Beschädigung der Sensorschicht 11. Die Sensorschicht 11 ist in diesem Ausführungsbeispiel 50 nm dick. Diese kann allerdings bis hinunter zu 10 nm Dicke mit entsprechender Funktionalität hergestellt werden. Es sind im Wesentlichen keine Querempfindlichkeiten gegenüber anderen Gasen im Gefahrenraum festzustellen. Dieses begründet sich dadurch, dass die Änderung der optischen Transmission der Sensorschicht bzw. des Kompositfilm's nur durch Veränderung der Kristallstruktur der Cluster 25, insbesondere Nanocluster, hervorgerufen wird, die ausschließlich unter Wasserstoffeinfluss zu beobachten ist.

Der Sensor weist eine hohe Lebensdauer auf, da die eigentlichen optisch schaltenden bzw. den optischen Effekt hervorrufenden Metall-Nanocluster in einer Polymermatrix eingebettet sind. Da die Matrix aus Polymeren besteht, kann diese den mechanischen Stress der Nanocluster absorbieren. Es erfolgt weder eine Degeneration des Nanokomposits wie auch der Gesamtstruktur. Damit kann eine hohe Lebensdauer erzielt werden und auch Messungen von Wasserstoffkonzentrationen von bis 10 Vol % und höher reproduzierbar können möglich werden. Aufgrund der rein optischen Funktionsweise ist ein 100%-iger Explosionsschutz auch im Gefahrenraum gegeben.

### Bezuaszeichenliste

- 10: Sensor
- 11: Sensorschicht
- 12: Substrat
- 13: Spiegelfläche
- 14: Halterung
- 15: Lichtwellenleiter
- 16: Lichtwellenleiter
- 177: Lichtquelle
- 18: Detektor
- 19: Kollimatorlinse
- 20: Umgebung
- 21: Gaszufuhrleitung
- 22: Pufferschicht
- 25: Cluster
- 26: Matrix
- D: Durchmesser

## Patentansprüche

1. Wasserstoffsensor (10) mit einer Strahlenquelle (17), mittels der elektromagnetische Strahlen auf ein Sensormedium (11) gestrahlt werden, wobei das Sensormedium (11) einen Transmissionskoeffizienten aufweist, der in Abhängigkeit der Konzentration von Wasserstoff in der Umgebung (20) des Sensormediums (11) variiert, und mit einem Detektor (18), der wenigtens einen Teil der durch das Sensormedium (11) transmittierten Strahlung detektiert, **dadurch gekennzeichnet, dass** das Sensormedium (11) Cluster (25) enthaltend oder bestehend aus einer Palladiumlegierung, Yttrium, Scandium, wenigstens einem Lanthanid, wenigstens einem Actinoid, Wolframoxid und/oder Vanadiumoxid und/oder eine Mischung oder Verbindung dieser Materialien aufweist, wobei die Cluster (25) in einer Matrix (26) aus einem Polymer eingebettet sind.

2. Wasserstoffsensor nach Anspruch 1, **dadurch gekennzeichnet, dass** die Cluster (25) einen Durchmesser (D) von 1 nm bis 30 nm, insbesondere 2 nm bis 15 nm, insbesondere 3 nm bis 10nm, haben.

3. Wasserstoffsensor nach Anspruch 1 oder 2, **dadurch gekennzeichnet, dass** das Polymer Polytetrafluorethylen, Polyterephthalat, Polyimid, Polymethylmethacrylat und/oder Polycarbonat umfasst.

4. Wasserstoffsensor nach einem der Ansprüche 1 bis 3, **dadurch gekennzeichnet, dass** das Sensormedium (11) auf einem Substrat (12) aufgebracht ist, wobei zwischen Substrat (12) und Sensormedium (11) eine Pufferschicht (22) vorgesehen ist.

5. Wasserstoffsensor nach einem der Ansprüche 1 bis 4, **dadurch gekennzeichnet, dass** auf dem Sensormedium (11) eine Deckschicht (23) vorgesehen ist, wobei insbesondere das Sensormedium (11) als Schicht ausgebildet ist.

6. Wasserstoffsensor nach einem der Ansprüche 1 bis 5, **dadurch gekennzeichnet, dass** ein Reflektor (12, 13) vorgesehen ist, der die durch das Sensormedium (11) transmittierten Strahlen zu dem Sensormedium (11) zurück reflektiert.

7. Wasserstoffsensor nach Anspruch 6, **dadurch gekennzeichnet, dass** das Substrat (12) der Reflektor ist oder ein Reflektor auf dem Substrat aufgebracht ist.

8. Wasserstoffsensor nach Anspruch 6 oder 7, **dadurch gekennzeichnet, dass** elektromagnetische Strahlen mit wenigstens einem ersten Lichtwellenleiter (15) von einer Strahlenquelle (17) zu dem Sensormedium (11) geleitet werden, wobei insbesondere von dem Reflektor (12, 13) reflektierte und durch das Sensormedium (11) transmittierte Strahlen mit wenigstens einem zweiten Lichtwellenleiter (16) zu dem Detektor (18) geleitet werden, wobei insbesondere der wenigstens eine erste und der wenigstens eine zweite Lichtwellenleiter (15, 16) wenigstens abschnittsweise im Wesentlichen parallel verlaufen.

9. Wasserstoffsensor nach Anspruch 8, **dadurch gekennzeichnet, dass** am zu dem Sensormedium (11) gerichteten Ende des wenigstens einen ersten und/oder zweiten Lichtwellenleiters (15, 16) eine die Strahlen bündelnde Linse (19) vorgesehen ist.

## Claims

1. A hydrogen sensor (10) with a radiation source (17), by means of which electromagnetic radiation is radiated onto a sensor medium (11), wherein the sensor medium (11) has a transmission coefficient which varies in dependence on the concentration of hydrogen in the vicinity (20) of the sensor medium (11), and including a detector (18), which detects at least a proportion of the radiation transmitted through the sensor medium (11), **characterised in that** the sensor medium (11) includes clusters (25) containing or consisting of a palladium alloy, yttrium, scandium, at least one lanthanide, at least one actinoid, tungsten oxide and/or vanadium oxide and/or a mixture or compound of these materials, wherein the clusters (25) are embedded in a matrix (26) of a polymer.

2. A hydrogen sensor as claimed in claim 1, **characterised in that** the clusters (25) have a diameter (D) of 1nm to 30nm, particularly 2nm to 15nm, particularly 3nm to 10nm.

3. A hydrogen sensor as claimed in claim 1 or 2, **characterised in that** the polymer includes polytetrafluoroethylene, polyterephthalate, polyimide, polymethylmethacrylate and/or polycarbonate.

4. A hydrogen sensor as claimed in one of claims 1 to 3, **characterised in that** the sensor medium is deposited on a substrate (12), wherein a buffer layer (22) is provided between the substrate (12) and sensor medium (11).

5. A hydrogen sensor as claimed in one of claims 1 to 4, **characterised in that** provided on the sensor medium (11) there is a cover layer (23), wherein, in particular, the sensor medium (11) is in the form of a layer.

6. A hydrogen sensor as claimed in one of claims 1 to 5, **characterised in that** a reflector (12, 13) is provided, which reflects the radiation transmitted through the sensor medium (11) back to the sensor medium (11).

7. A hydrogen sensor as claimed in claim 6, **characterised in that** the substrate (12) is the reflector or a reflector is deposited on the substrate.

8. A hydrogen sensor as claimed in claim 6 or 7, **characterised in that** electromagnetic radiation is conducted with at least one first optical wave guide (15) from a radiation source (17) to the sensor medium (11), wherein, in particular, radiation reflected by the reflector (12, 13) and transmitted through the sensor medium (11) is conducted with at least one second optical wave guide (16) to the detector (18), wherein, in particular, the at least one first and the at least one second optical wave guide (15, 16) extend, at least in sections, substantially parallel.

9. A hydrogen sensor as claimed in claim 8, **characterised in that** a lens (19), which focuses the radiation, is provided at the end directed towards the sensor medium (11) of the at least one first and/or second optical wave guide (15, 16).

## Revendications

1. Capteur d'hydrogène (10) comprenant une source de rayonnement (17), à l'aide de laquelle des rayons électromagnétiques sont envoyés sur un milieu capteur (11), le milieu capteur (11) ayant un coefficient de transmission qui varie en fonction de la concentration de l'hydrogène dans l'environnement (20) du milieu capteur (11), ainsi qu'un détecteur (18), qui détecte au moins une partie du rayonnement transmise à travers le milieu capteur (11), **caractérisé en ce que** le milieu capteur (11) comprend des agrégats (25), contenant, ou en étant constitués, un alliage de palladium, de l'yttrium, du scandium, au moins un lanthanide, au moins un actinide, de l'oxyde de tungstène et/ou de l'oxyde de vanadium et/ou un mélange ou une combinaison de ces matériaux, les agrégats (25) étant incorporés dans une matrice (26) constituée d'un polymère.

2. Capteur d'hydrogène selon la revendication 1, **caractérisé en ce que** les agrégats (25) ont un diamètre (D) de 1 à 30 nm, en particulier de 2 à 15 nm, en particulier de 3 à 10 nm.

3. Capteur d'hydrogène selon la revendication 1 ou 2, **caractérisé en ce que** le polymère comprend du polytétrafuoréthylène, un polytéréphtalate, du polyimide, du poly(méthacrylate de méthyle) et/ou un polycarbonate.

4. Capteur d'hydrogène selon l'une des revendications 1 à 3, **caractérisé en ce que** le milieu capteur (11) est appliqué sur un substrat (12), une couche tampon (22) étant prévue entre le substrat (12) et le milieu capteur (11).

5. Capteur d'hydrogène selon l'une des revendications 1 à 4, **caractérisé en ce qu'**il est prévu sur le milieu capteur (11) une couche de couverture (23), le milieu capteur (11) étant configuré en particulier sous forme d'une couche.

6. Capteur d'hydrogène selon l'une des revendications 1 à 5, **caractérisé en ce qu'**un réflecteur (12, 13) est prévu, qui renvoie par réflexion vers le milieu capteur (11) les rayons transmis par le milieu capteur (11).

7. Capteur d'hydrogène selon la revendication 6, **caractérisé en ce que** le substrat (12) est le réflecteur, ou un réflecteur est appliqué sur le substrat.

8. Capteur d'hydrogène selon la revendication 6 ou 7, **caractérisé en ce que** des rayons électromagnétiques sont, à l'aide d'au moins un guide d'ondes lumineuses (15), guidés d'une source de rayonnement (17) à un milieu capteur (11), les rayons réfléchis par le réflecteur (12, 13) et transmis par le milieu capteur (11) étant, avec au moins un deuxième guide d'ondes lumineuses (16), en particulier guidés vers le détecteur (18), le ou les premiers et deuxièmes guides d'ondes lumineuses (15, 16) étant, en particulier, au moins par tronçons pour l'essentiel parallèles.

9. Capteur d'hydrogène selon la revendication 8, **caractérisé en ce qu'**il est prévu, au niveau de l'extrémité, dirigée vers le milieu capteur (11), du ou des premiers et/ou deuxièmes guides d'ondes lumineuses (15, 16), une lentille (19) qui concentre les rayons.
